# EUROPÄISCHE PATENTSCHRIFT

(11) **EP 0 756 499 B1**
(45) Veröffentlichungstag und Bekanntmachung des Hinweises auf die Patenterteilung: **07.08.2002**
(21) Anmeldenummer: 96901711.0
(22) Anmeldetag: 06.02.1996
(51) Int. Cl.: A61L 27/00, C25D 9/04

(54) **VERFAHREN ZUR HERSTELLUNG EINER GRADIERTEN BESCHICHTUNG AUS CALCIUMPHOSPHATPHASEN UND METALLOXIDPHASEN AUF METALLISCHEN IMPLANTATEN**
PROCESS FOR PRODUCING A GRADUATED COATING OF CALCIUM PHOSPHATE PHASES AND METALLIC OXIDE PHASES ON METAL IMPLANTS
PROCEDE POUR PRODUIRE UN REVETEMENT A GRADIENTS DE PHASES DE PHOSPHATE DE CALCIUM ET DE PHASES D'OXYDE METALLIQUE SUR DES IMPLANTS METALLIQUES

(30) Priorität: 10.02.1995 DE 19504386
(43) Veröffentlichungstag der Anmeldung: 05.02.1997
(73) Patentinhaber: Biomet Merck GmbH, 6460 Altdorf (CH)
(72) Erfinder: SCHARNWEBER, Dieter, D-01324 Dresden (DE); BERSCH, Henrike, D-01097 Dresden (DE); WORCH, Hartmut, D-01069 Dresden (DE); HOFINGER, Jürgen, D-01309 Dresden (DE); KRANZ, Curt, D-10825 Berlin (DE); POMPE, Wolfgang, D-01737 Kurort Hartha (DE)
(74) Vertreter: Eiermann, Volker
(86) Internationale Anmeldenummer: DE9600197
(87) Internationale Veröffentlichungsnummer: WO9624391

(56) Entgegenhaltungen:
- CA-A- 2 073 781

## Beschreibung

Die Erfindung betrifft ein Verfahren zur Herstellung einer gradierten Beschichtung mit Calciumphosphatphasen und Metalloxidphasen auf metallischen Implantaten, insbesondere aus Titan oder Titanlegierungen, für die Verwendung als Dental-, Kiefer- oder Gelenkimplantat.
Es ist bekannt, daß der Zeitraum des Einwachsens bis zum Vorliegen einer mechanischen Belastbarkeit eines Implantats verkürzt werden kann, wenn ein metallisches Implantat verwendet wird, das mit Calciumphosphatphasen und insbesondere mit dem dem Knochenmineral entsprechenden Calciumphosphat, dem Hydroxylapatit, beschichtet ist.

Der Verbund zwischen Metallimplantat und Calciumphosphaten wird auf verschiedene Weise realisiert. Nach EP-A 0006544 werden die kugelförmigen Calciumphosphatpartikel in die Oberfläche durch Ausgießen einer Form, mit dem Implantatmetall eingebracht. In US-PS 4.145.764 wird ein Verfahren beschrieben, bei dem Keramikteilchen thermisch auf das Implantat aufgespritzt werden. Diese Verfahren sind jedoch sehr energieintensiv, kosten- und zeitaufwendig. Aus EP 0232791 und EP 0237053 sind weiterhin Verfahren bekannt, bei denen durch anodische Oxidation unter Funkenentladung in wäßrigen Elektrolyten eine in Oxiden enthaltene, resorbierbare Calciumphosphatkeramik auf Titan aufgebracht wird. Die dabei erzeugten Schichten bestehen aber nicht aus Hydroxylapatit oder Fluorapatit, sondern aus Oxiden und stark resorbierbaren Calciumphosphaten. Mit der vollständigen Resorption der Calciumphosphatphasen geht dabei auch der bioaktive Charakter des Implantats verloren.

CA 2,073,781 A1 betrifft ein Verfahren zur Bildung einer zweischichtigen Kompositbeschichtung auf einer konduktiven Implantateinrichtung. Beginnend mit einer anodischen Polarisierung führt der mehrfache Wechsel zwischen anodischer und kathodischer Polarisierung zu einer Beschichtung mit zwei verschiedenen Schichten, einer oxidischen Basisschicht und einer Kalziumphosphataußenschicht.

Die DE 43 03 575 C1 beschreibt ein Verfahren zur Herstellung apatitbeschichteter Metallimplantate unter Induzierung einer plasmachemischen Reaktion mittels Wechselstrom in wäßrigen Lösungen. Als Elektrolyt wird dabei eine Lösung aus Alkali- oder Erdalkalisalzen verwendet, in der Hydroxylapatit und/oder Flurapatit mit einer definierten Körnung und Konzentration dispergiert ist. Das plasmachemische Verfahren führt zu Schichten, die aus bis zu 95% reinem Hydroxylapatit oder Fluorapatit bestehen.
Nachteile des Verfahrens liegen insbesondere in den auftretenden großen Schichtdicken (bis 250 µm) sowie in der groben Körnigkeit der Schichten (bis zu 100 µm). zudem ist die Haftung der Schichten nicht optimal.

Die WO 92/13984 beschreibt ein Verfahren zur Abscheidung bioaktiver Schichten auf leitfähigen Substraten. In einer Elektrolysezelle ist eine inerte Anode und eine Elektrolytlösung enthalten, die aus einer wäßrigen Lösung von Ionen der Keramik besteht und einen pH-Wert von weniger als 8 aufweist. Das aktivierte leitfähige Substrat wird in die Elektrolytlösung eingetaucht und das Potential zwischen Anode und leitfähigem Substrat so eingestellt, daß durch eine pH-Wert-Erhöhung an der Nahtstelle zwischen Elektrolytlösung und leitfähigem Substrat eine keramische Schicht auf dem leitfähigen Substrat abgeschieden wird.

Nachteilig an der Lösung ist, daß die Schichtabscheidung nur auf der Oberfläche des Substrats erfolgt, so daß zum einen keine belastbare Verbindung zu dieser entstehen kann und die Schicht zum anderen biologisch vollständig resorbierbar ist. Weiterhin besteht die abgeschiedene Schicht prinzipiell im wesentlichen aus drei Bestandteilen, α- und β-Tricalciumphosphat und Komponenten der Formel Ca₅(PO₄)₃₋ₓ(CO₃)ₓ(OH)₁₊ₓ mit einem Wert von x = 0.2 und weniger. Die Schicht stellt somit stets eine Mischung dar, die dem strukturellen Aufbau des Knochens nicht nahe kommt.

Die Aufgabe der Erfindung besteht darin, metallische Implantate vorzugsweise aus Titan oder Titanlegierungen mit einer gradierten Beschichtung aus Calciumphosphatphasen und Metalloxidphasen zu beschichten, so daß neben einer Beschleunigung des Einwachsverhaltens eine dauerhafte Verbesserung der Wechselwirkung zwischen Implantatoberfläche und dem biologischen System erreicht wird. Daneben soll die Zusammensetzung der Calciumphosphatphasen über Verfahrensparameter steuerbar sein, so daß wahlweise Hydroxylapatit, Octacalciumphosphat oder Brushit bzw. definierte Mischungen aus diesen Phasen erzeugt werden können.

Erfindungsgemäß wird die Aufgabe durch ein elektrochemisches Verfahren mit einer vom Metallimplantat gebildeten Substratelektrode und einer Gegenelektrode gelöst, bei
dem als Elektrolyt eine wässrige Lösung mit Calcium- und Phosphationen im schwach sauren bis etwa neutralen Bereich verwendet und die vom Implantat gebildete Substratelektrode beginnend mit einer kathodischen Polarisation mehrfach wechselnd kathodisch und anodisch polarisiert wird.

Vorzugsweise wird das Konzentrationsverhältnis an Calcium- und Phosphationen im Elektrolyten so gewählt, daß es dem Konzentrationsverhältnis von Calcium- und Phosphationen in Hydroxylapatit entspricht. In einer bevorzugten Ausführungsform der Erfindung wird der Elektrolyt aus einer wässrigen CaCl₂ - und einer NH₄H₂PO₄-Lösung mit einer dem Hydroxylapatit entsprechenden Konzentration an Calcium- und Phosphationen hergestellt. Der pH-Wert wird vorzugsweise mit verdünnter NH₄OH-Lösung auf einen Wert von 4 bis 7,5 eingestellt. Die Verwendung anderer leicht löslicher Calciumsalze sowie Phosphate (z.B. Alkaliphosphate) ist ebenfalls möglich. Darüber hinaus sind es wesentlich die Elektrolysebedingungen, die die Bildung von bestimmten Calciumphosphatphasen bzw. derer Mischungen bestimmen. Damit ist es auch möglich, andere Konzentrationsverhältnisse an Calcium- und Phosphationen zu wählen.

In einer aus Substratelektrode, Bezugselektrode und Gegenelektrode gebildeten Anordnung wird die vom Implantat gebildete Substratelektrode wechselnd kathodisch und anodisch polarisiert. Vorzugsweise wird die Substratelektrode in einem ersten Schritt kathodisch polarisiert. Im mehrfachen Wechsel schließen sich anodische und kathodische Polarisation an, wobei vorzugsweise die Abscheidung bei kathodischer Polarisation der Substratelektrode beendet wird.
Vorteilhaft werden die Zeiten für die kathodische und anodische Polarisation bei ihrer Wiederholung erhöht und/oder die anodische Polarisation mit positiv werdendem anodischen Potential durchgeführt. Kathodische und anodische Polarisation der Substratelektrode erfolgen im Wechsel mit einer Gesamtdauer der kathodischen Polarisation zwischen 1 und 60 min und einer Gesamtdauer der anodischen Polarisation zwischen 1 und 60 min.

Die kathodische Polarisation kann potentiostatisch oder galvanostatisch und die anodische Polarisation potentiostatisch, potentiodynamisch oder galvanostatisch bis zum Erreichen eines gewünschten Zielpotentials geführt werden. Das Zielpotential liegt im Bereich von 2 bis 150 V_{SCE}. Die Wahl des Zielpotentials bestimmt dabei die Dicke der auszubildenden Titanoxidschicht und damit letztlich auch die des gradierten Bereichs der Beschichtung.

Die Stromdichte der kathodischen galvanostatischen Polarisation wird vorzugsweise auf etwa 0.1 bis 5 mA/cm² eingestellt

Die Drei-Elektroden-Anordnung zur Durchführung des erfindungsgemäßen Verfahrens ist aus gesättigter Kalomelelektrode als Bezugselektrode, Platinblech als Gegenelektrode und Metallimplantat als Substratelektrode aufgebaut. Als Elektrolytzelle wird eine thermostatierbare Zelle verwendet. Die elektrochemische Reaktion wird vorzugsweise bei etwa 60 °C durchgeführt.

Mit Hilfe des erfindungsgemäßen Verfahrens läßt sich eine gewünschte Calciumphosphatphase bzw. eine definierte Mischung aus Calciumphosphatphasen als gradiertes Gefüge mit dem Metalloxid des Implantatmaterials aus der verwendeten Elektrolytlösung auf dem Metallimplantat ausbilden. Die auf dem Metallimplantat abgeschiedene Schicht bewirkt ein gutes Heranwachsen des Knochens an das Implantat. Damit wird der Einbau des Implantats in das biologische System befördert. Unter Einwirkung körpereigener Substanzen wird die Schicht normalerweise aufgebraucht. Um dem Knochen eine Orientierung über die gesamte Lebensdauer zu geben, werden Calciumphosphatpartikel nicht nur fest mit dem Implantat verbunden, sondern in diesem fest eingebaut. Dies gelingt durch die schrittweise kathodische und anodische Polarisation der Substratelektrode. An dieser wird bei kathodischer Polarisation die gewünschte Calciumphosphatphase bzw. eine definierte Mischung aus Phasen aus dem Elektrolyten angelagert. Bei anodischer Polarisation beginnt die Oxidschicht des Implantatmetalls über die angelagerten Calciumphosphatpartikel hinauszuwachsen, wodurch diese in die Oxidschicht des Metalls eingelagert werden. Durch Wiederholung der beiden Schritte wird die Dicke der Schicht erhöht. Für das Wachstum der Schicht aus Calciumphosphatphasen ist es vorteilhaft, die Zeiten für die Schritte zu erhöhen und den zweiten Schritt mit positiver werdendem anodischen Potential durchzuführen. Damit die äußere Schicht durch Calciumphosphatphasen gebildet wird, ist ein Abschluß der Bildung der gradierten Schicht mit kathodischer Polarisation vorteilhaft.

Der Vorteil der erfindungsgemäß abgeschiedenen Schichten besteht darin, daß durch den festen Einbau der Calciumphosphatphasen in die Implantatoberfläche eine verbesserte Kraftübertragung und eine dauerhafte Verbesserung der Biokompatibilität erreicht werden. Durch die Möglichkeit der Anpassung der Zusammensetzung der Schicht an die Zusammensetzung der anorganischen Knochensubstanz wird ein schnelles Einwachsen in den Knochen begünstigt.
Anhand nachfolgender Ausführungsbeispiele wird die Erfindung näher erläutert.

### Ausführungsbeispiel 1

Eine Scheibe aus 99.7 % reinem Titan mit einem Durchmesser von 13 mm, einer Dicke von 2 mm wird geschliffen, in Alkohol gereinigt, mit deionisiertem Wasser gespült und föngetrocknet. Als Elektrolytlösung dient eine Calciumphosphatlösung, die folgendermaßen hergestellt wird: Jeweils 10 ml Stammlösung von CaCl₂ · 2H₂O und NH₄H₂PO₄ in den Konzentrationen 33 mM und 20 mM werden verdünnt und gemischt, so daß 200 ml entstehen; 1.67 mM an Calcium- und 1.0 mM an Phosphationen. Der pH-Wert wird zuvor mit verdünnter NH₄OH-Lösung auf 6.4 eingestellt. Die Lösung wird auf 60 °C erhitzt und in eine Doppelmantelzelle gegeben. Eine Drei-Elektroden-Anordnung wird aufgebaut. Als Bezugselektrode dient eine gesättigte Kalomelelektrode, Gegenelektrode ist ein Platinblech und Arbeitselektrode ist die Titanscheibe. Die Kontaktierung des Potentiostaten wird vorgenommen. Die Ausbildung der Hydroxylapatitschicht erfolgt über eine wechselnde Polarisation:
10 min kathodische Polarisation der Titanprobe, galvanostatisch mit I=1 mA (1. Schritt);
10 min anodische Polarisation, potentiostatisch mit U = 5 V_{SCE} (2. Schritt);
15 min kathodische Polarisation wie im 1. Schritt (3. Schritt);
10 min anodische Polarisation mit U = 10 V_{SCE} (4. Schritt);
abschließend 35 min kathodische Polarisation wie im 1. Schritt (5. Schritt).

Die Probe wird aus dem Elektrolysebad genommen, mit deionisiertem Wasser gespült und föngetrocknet. Die abgeschiedene Schicht erscheint weißlich gelblich, ist einheitlich ausgebildet und haftet gut. Die elektronenmikroskopische Untersuchung zeigt eine geschlossene Schicht, die aus Agglomeraten kleinster Nadeln besteht (Länge der Nadeln etwa 500 nm). Die Analyse der Zusammensetzung mittels energiedispersiver Röntgenanalyse liefert ein Ca/P-Verhältnis der Phase in der Schicht, das dem von kommerziellen Hydroxylapatit entspricht Röntgenbeugungsuntersuchungen erbringen mit Hilfe der Kristallstruktur den Nachweis der Phase Hydroxylapatit.
Die Schrägpräparation zeigt eine Gradierung der Phasengrenze, elektronenmikroskopisch ist kein scharfer Übergang zwischen Substrat und Schicht zu beobachten.

### Ausführungsbeispiel 2

Ein Elektrolyt identisch dem aus Beispiel 1 wird verwendet. Die Titanscheibe wird wie in Beispiel 1 hergestellt. Der elektrochemische Aufbau entspricht dem in Beispiel 1. Nach einer galvanostatischen kathodischen Polarisation (I = 0.3 mA, 10 min) (Schritt 1) wird im 2. Schritt ausgehend von dem sich bei der galvanostatischen kathodischen Polarisation einstellenden Potential mit einer Polarisationsgeschwindigkeit von 5 mV/s anodisch bis auf 5 V_{SCE} polarisiert Nach Erreichen des Potentials wird in Schritt 3 15 min eine kathodische Polarisation mit 0.3 mA durchgeführt. Als 4. Schritt wird ausgehend von dem sich bei der galvanostatischen kathodischen Polarisation einstellenden Potential mit einer Polarisationsgeschwindigkeit von 5 mV/s anodisch bis auf 10 V_{SCE} polarisiert Abschließend erfolgt 35 min kathodische Polarisation wie im 1. Schritt (5. Schritt).
Die auf der Substratelektrode vorliegende Schicht besteht aus Octacalciumphosphat. Die elektronenmikroskopische Untersuchung zeigt nadel- bis bänderförmige Kristalle mit einer Ausdehung bis zu 5 µm. Vergleichende röntgendiffraktometrische und ramanspektroskopische Untersuchungen liefern den Nachweis, daß die Schicht aus Octacalciumphosphat aufgebaut ist.
Die Schrägpräparation zeigt eine Gradierung der Phasengrenze, elektronenmikroskopisch ist kein scharfer Übergang zwischen Substrat und Schicht zu beobachten.

### Ausführungsbeispiel 3

Eine Titanscheibe wird wie in Beispiel 1 hergestellt. Als Elektrolytlösung werden 200 ml einer Calciumphosphatlösung verwendet, die durch Einwaage der Salze wie folgt zusammengesetzt ist: 40 mM CaCl₂ und 25 mM NH₄H₂PO₄. Der pH-Wert wird auf 4.4 eingestellt.
Der elektrochemische Aufbau entspricht dem in Beispiel 1.
Nach der 1. potentiostatischen kathodischen Polarisation mit U_{SCE} = -1300 mV, 10 min (Schritt 1) wird die Elektrode im 2. Schritt 10 min mit 1mA galvanostatisch anodisch polarisiert. Nach einer 15 minütigen kathodischen Polarisation wie in Schritt 1 (3. Schritt) wird im 4. Schritt 10 min mit 2 mA galvanostatisch anodisch polarisiert. Abschließend erfolgt eine 35 minütige kathodische Polarisation wie in Schritt 1 (5. Schritt).
Die auf der Substratelektrode vorliegende Schicht besteht aus plättchenförmigen Kristallen mit einer Ausdehung bis zu 30 µm.
Die energiedispersive Röntgenanalyse liefert ein Ca/P-Verhältnis der Phase in der Schicht, das dem von kommerziellen Brushit entspricht. Röntgenbeugungsuntersuchungen erbringen mit Hilfe der Kristallstruktur den Nachweis der Phase Hydroxylapatit.
Die Schrägpräparation zeigt eine Gradierung der Phasengrenze, elektronenmikroskopisch ist kein scharfer Übergang zwischen Substrat und Schicht zu beobachten.

### Ausführungsbeispiel 4

Ein Elektrolyt identisch dem aus Beispiel 1 wird verwendet. Die Titanscheibe wird wie in Beispiel 1 hergestellt und zusätzlich bis zu einer spiegelnden Oberfläche poliert und in 50 ml einer Lösung, die 0.5 M an NaOH und 0.1 M an H₂O₂ ist, 4 min lang bei 65 °C oxidierend geätzt. Die geätzte Oberfläche läßt mittels Rasterelektronenmikroskop einen unterschiedlichen Angriff der Titankristallite und eine Strukturierung im nm-Maßstab erkennen. Die Abscheidung der Schicht erfolgt in gleicher Weise wie in Ausführungsbeispiel 1. Die elektronenmikroskopische Untersuchung zeigt nadelförmige Kristallite wie in Beispiel 1 mit einem Ca/P-Verhältnis für Hydroxylapatit. Insgesamt weist die Schicht eine größere Rauhigkeit auf. Die Schicht zeigt eine bessere Haftung auf der Unterlage als die in Beispiel 1 hergestellte. Der untersuchte Schrägschliff zeigt zusätzlich zur Gradierung eine Verzahnung zwischen Substrat und Schicht.

## Patentansprüche

1. Verfahren zur Herstellung einer gradierten Beschichtung mit Calciumphosphatphasen und Metalloxidphasen auf metallischen Implantaten mit Hilfe elektrochemischer Reaktionen in wäßrigen Dispersionen, mit einer aus dem Metallimplantat gebildeten Substratelektrode und einer Gegenelektrode, wobei als Elektrolyt eine Lösung verwendet wird, in der Calcium- und Phosphationen vorliegen, **gekennzeichnet dadurch, daß** der pH-Wert der Lösung vorzugsweise im schwach sauren bis etwa neutralen Bereich eingestellt ist und die Substratelektrode beginnend mit einer kathodischen Polarisation mehrfach wechselnd kathodisch und anodisch polarisiert und daß beim Wechsel von kathodischer und anodischer Polarisation im Folgeschritt die Zeitdauer der Polarisation erhöht und/oder die anodische Polarisation mit positiver werdendem Potential durchgeführt wird.

2. Verfahren nach Anspruch 1, **gekennzeichnet dadurch, daß** das Konzentrationsverhältnis von Calcium- und Phosphationen im Elektrolyten dem in Hydroxylapatit entspricht.

3. Verfahren nach Anspruch 1, **gekennzeichnet dadurch, daß** der pH-Wert der Elektrolytlösung 4 bis 7,5 beträgt.

4. Verfahren nach einem der Ansprüche 1 bis 3, **gekennzeichnet dadurch, daß** die Substratelektrode beginnend mit einer kathodischen Polarisation mehrfach wechselnd kathodisch und anodisch polarisiert und die Abscheidung mit einer kathodischer Polarisation beendet wird.

5. Verfahren nach Anspruch 1, **gekennzeichnet dadurch, daß** die kathodische Polarisation potentiostatisch oder galvanostatisch und die anodische Polarisation potentiostatisch, potentiodynamisch oder galvanostatisch bis zum Erreichen eines im Bereich von 2 bis 150 V_{SCE} liegenden Zielpotentials erfolgt.

6. Verfahren nach Anspruch 1, **gekennzeichnet dadurch, daß** kathodische und anodische Polarisation im Wechsel und mit einer Gesamtdauer der kathodischen Polarisation zwischen 1 und 60 min und mit einer Gesamtdauer der anodischen Polarisation zwischen 1 und 60 min erfolgen.

7. Verfahren nach Anspruch 1, **gekennzeichnet dadurch, daß** die Stromdichte der kathodischen galvanostatischen Polarisation 0.1 bis 5 mA/cm² beträgt.

8. Metallisches Implantat, herstellbar nach einem Verfahren gemäß der Ansprüche 1 bis 7, **dadurch gekennzeichnet, dass** das Implantat eine gradierte Beschichtung aus Calciumphosphatphasen und Metalloxidphasen aufweist.

9. Verwendung eines metallischen Implantates nach Anspruch 8 zur Herstellung eines Dental-, Kiefer- oder Gelenkimplantates.

## Claims

1. Process for producing a gradient coating with calcium phosphate phases and metal oxide phases on metallic implants by means of electrochemical reactions in aqueous dispersions using a substrate electrode formed from the metal implant and a counterelectrode, wherein the electrolyte used is a solution containing calcium and phosphate ions, **characterized in that** the pH of the solution has preferably been set in the weakly acidic to approximately neutral range and the substrate electrode, starting with a cathodic polarization, is repeatedly and alternately polarized cathodically and anodically and **in that** in the course of changing from cathodic and anodic polarization the next step is carried out with an increased polarization time and/or with an increasingly positive potential for the anodic polarization.

2. Process according to Claim 1, **characterized in that** the concentration ratio of calcium and phosphate ions in the electrolyte is equal to that in hydroxyapatite.

3. Process according to Claim 1, **characterized in that** the pH of the electrolyte solution is 4 to 7.5.

4. Process according to any of Claims 1 to 3, **characterized in that** the substrate electrode, starting with a cathodic polarization, is repeatedly and alternately polarized cathodically and anodically and the deposition is terminated with a cathodic polarization.

5. Process according to Claim 1, **characterized in that** the cathodic polarization is carried out potentiostatically or galvanostatically and the anodic polarization is carried out potentiostatically, potentiodynamically or galvanostatically until a target potential in the range from 2 to 150 V_{SCE} is reached.

6. Process according to Claim 1, **characterized in that** cathodic and anodic polarization are effected alternately and with a total time between 1 and 60 min for the cathodic polarization and between 1 and 60 min for the anodic polarization.

7. Process according to Claim 1, **characterized in that** the current density of the cathodic galvanostatic polarization is 0.1 to 5 mA/cm².

8. Metallic implant obtainable by a process according to Claims 1 to 7, **characterized in that** the implant comprises a gradient coating of calcium phosphate phases and metal oxide phases.

9. Use of a metallic implant according to Claim 8 for producing a dental, maxillofacial or joint implant.

## Revendications

1. Procédé pour la réalisation d'un revêtement gradué comportant des phases phosphates de calcium et des phases oxydes métalliques sur des implants métalliques au moyen de réactions électrochimiques dans des dispersions aqueuses, avec une électrode substrat formée par l'implant métallique et une contre-électrode, une solution dans laquelle se trouvent des ions calcium et des ions phosphates étant utilisée comme électrolyte, **caractérisé en ce que** l'on ajuste le pH de la solution, de préférence dans un intervalle allant d'une acidité faible à approximativement la neutralité et **en ce que** l'on polarise cathodiquement et anodiquement l'électrode substrat alternativement plusieurs fois et en commençant par une polarisation cathodique, et **en ce que** l'on augmente la durée de la polarisation lors du changement des polarisations cathodique et anodique dans l'étape ultérieure et/ou **en ce que** l'on effectue la polarisation anodique avec un potentiel devenant positif.

2. Procédé selon la revendication 1, **caractérisé en ce que** le rapport des concentrations ions calclum/ions phosphates dans l'électrolyte correspond à celui que l'on trouve dans l'hydroxyapatite.

3. Procédé selon la revendication 1, **caractérisé en ce que** le pH de la solution électrolytique est compris entre 4 et 7,5.

4. Procédé selon l'une des revendications 1 à 3, **caractérisé en ce que** l'on polarise cathodiquement et anodiquement l'électrode substrat alternativement plusieurs fois et en commençant par une polarisation cathodique et **en ce que** l'on achève la précipitation avec une polarisation cathodique.

5. Procédé selon la revendication 1, **caractérisé en ce que** la polarisation cathodique potentiostatique ou galvanostatique et la polarisation anodique potentiostatique, potentiodynamique ou galvanostatique sont effectuées jusqu'à l'obtention d'un potentiel cible situé dans un intervalle allant de 2 à 150 V_{SCE}.

6. Procédé selon la revendication 1, **caractérisé en ce que** l'on effectue la polarisation cathodique et la polarisation anodique en alternance et avec une durée totale de la polarisation cathodique comprise entre 1 et 60 min et avec une durée totale de la polarisation anodique comprise entre 1 et 60 min.

7. Procédé selon la revendication 1, **caractérisé en ce que** la densité de courant de la polarisation cathodique galvanostatique est comprise entre 0,1 et 5 mA/cm².

8. Implant métallique pouvant être obtenu par un procédé selon les revendications 1 à 7, **caractérisé en ce que** l'implant présente un revêtement gradué constitué par des phases phosphates de calcium et des phases oxydes métalliques.

9. Utilisation d'un implant métallique selon la revendication 8 pour la fabrication d'un implant dentaire, maxillaire ou articulaire.
